(19) Europäisches Patentamt

**Europäisches Patentamt**

**European Patent Office**

**Office européen des brevets**

(11) Veröffentlichungsnummer: **0 200 792**
**B1**

(12) **EUROPÄISCHE PATENTSCHRIFT**

(45) Veröffentlichungstag der Patentschrift: 09.05.90

(21) Anmeldenummer: 85101081.9

(22) Anmeldetag: 01.02.85

(51) Int. Cl.⁵: **C 12 M 1/02, C 12 M 1/12**

(54) **Folienfermenter.**

(43) Veröffentlichungstag der Anmeldung:
**12.11.86 Patentblatt 86/46**

(45) Bekanntmachung des Hinweises auf die
Patenterteilung: **09.05.90 Patentblatt 90/19**

(84) Benannte Vertragsstaaten:
**CH DE FR GB LI**

(56) Entgegenhaltungen:
**GB-A- 855 644**
**US-A-3 796 639**
**US-A-4 296 205**

(73) Patentinhaber: **Märkl, Herbert, Prof. Dr.-Ing.**
**Kleckener Kirchweg 34**
**D-2105 Seevetal 1 (DE)**

(72) Erfinder: **Märkl, Herbert, Prof. Dr.-Ing.**
**Kleckener Kirchweg 34**
**D-2105 Seevetal 1 (DE)**

(74) Vertreter: **Steinmann, Otto C. et al**
**Kanzlei Münich, Steinmann, Schiller**
**Willibaldstrasse 36**
**D-8000 München 21 (DE)**

Courier Press, Leamington Spa, England.

**Beschreibung**

Die Erfindung bezieht sich auf einen sterilisierbaren Fermenter für die Anzucht von Mikroorganismen oder von Zell- bzw. Gewebekulturen gemäß dem Oberbegriff des Anspruchs 1.

Ein Fermenter gemäß dem Oberbegriff des Anspruchs 1 ist in der US-PS 3 796 639 vorgeschlagen worden. Dieser sterilisierbare Fementer weist einen zylindrischen Reaktionsraum auf, den ein zylindrisches Kunststoffteil und zwei Stirnplatten begrenzen, die Einbauten für den Reaktionsraum halten. Das zylindrische Kunststoffteil ist ein "massives Teil" mit einer vergleichsweise großen Wandstärke.

Sterilisierbare Fermenter werden üblicherweise aus Edelstahl hergestellt. Die Festigkeit des Behälters hat sich dabei nach den Sterilisationsbedingungen (Sattdampf Wasser 121°C, 1,2 bar Überdruck, 0,5 h) zu richten. Aus Sicherheitsgründen empfiehlt die DECHEMA-Betreibernorm ("Arbeitsmethoden für die Biotechnologie", November 1982) eine Auslegungstemperatur von 143°C und einen Auslegungsdruck von 4 bar. Die Innenflächen sind möglichst reinigungs-freundlich auszubilden und auf Korn 240 zu bearbeiten.

Für Laborzwecke werden auch sogenannte "Glasfermenter" (übliche Reaktorgrößen 2 bis 200 l) eingesetzt, wie sie ebenfalls in der bereits genannten US-PS 3 796 639 angesprochen sind. Ein derartiger Fermenter besteht aus einem serienmäßig erhältlichen Glasrohr (Fa. Schott, Quickfit) mit einem Boden und einem Deckel aus Edelstahl. Alle Antriebselemente, Meßinstrumente sowie Zu- und Abführleitungen werden durch diese Stahlteile geführt.

Verglichen mit Ganzstahlfermentern haben Glasfermenter den Vorteil der visuellen Beobachtungsmöglichkeit. Darüberhinaus können Glasfermenter auch für die Anzucht photosynthetischer Kulturen eingesetzt werden, da der Reaktorinhalt von außen leicht zu beleuchten ist. Nachteilig ist ein hohes Sicherheitsrisiko bei der "in situ"-Sterilisation. (Unter "in situ"-Sterilisation versteht man eine Sterilisation des gefüllten Fermenters ohne Autoklaven.) Ein bei 121°C mit Innendruck belasteter Glasbehälter kann bereits bei geringfügigen Verletzungen des Glasmantels bersten. Die heute angebotenen Berstschutzmäntel, die für die Sterilisation verwendet werden, bieten aber keinen ausreichenden Schutz gegen freiwerdende Glassplitter sowie den Heißdampf (vgl. ACHEMA-Bericht "Biotechnologie", Chem.-Ing.-Techn. 54 (1982) Nr. 12, S. 1132-1138, insb. S. 1135).

Es wäre jedoch wünschenswert, über einen klar durchsichtigen Fermenter verfügen zu können, der die Verwendung genormter Meßsonden und anderer Einbauten, wie Rührorgane etc. zuläßt, und der bei Bedarf "in situ" sterilisierbar ist. Darüberhinaus sollten der Fermenter leicht zu reinigen, und die volumenbezogene Kosten möglichst gering sein.

Es sind zwar bereits Behälter für biologische Reaktionen etc vorgeschlagen worden, bei denen als Behältermaterial durchsichtige Folien verwendet werden, diese "Behälter" sind jedoch keine Fermenter im eigentlichen Sinne, da sie weder den Einbau der im Bereich der Fermentationstechnik üblichen Meßsonden und anderer Funktionselemente zulassen, noch bei Bedarf "in situ" sterilisierbar sind:

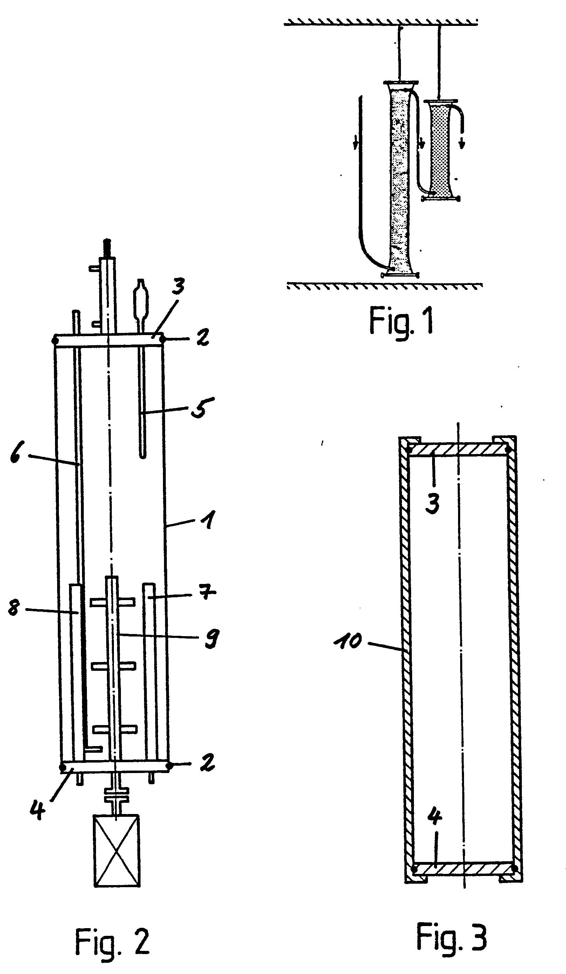

Beispielsweise ist ein Behälter bekannt (vgl. "Algae Biomass", G. Sheleff and C. J. Soeder, Editors, 1980 Elsevier, North-Holland Biomedical Press, S. 307-313, insb. S. 308), der - wie Fig. 1 zeigt - aus einem Folienschlauch (Polyethylen) mit einem Durchmesser von 30 cm und einer Länge von 1 bzw 2 m besteht Die Wandstärke des Folienschlauches beträgt 0,3 mm beim kleineren und 0,6 mm beim größeren Behälter (Doppelfolie). Der Folienschlauch ist durch Zusammendrükken längs einer geraden Linie verschlossen und wird an der oberen Verschlußklemme über ein Seil an der Raumdecke aufgehängt. Im jeweils oberen bzw. unteren Bereich des Kulturbehälters kann über einen eingeklebten Polyethylenschlauch dem Behälterinnenraum wahlweise Flüssigkeit oder Gas zugeführt bzw. entnommen werden. Weitere Einbauten sind nicht vorgesehen. Die angegebenen Kulturbehälter dienen der nichtsterilen Anzucht photoautotropher Mikroalgen bzw. von Rädertierchen (rotifers). Dieser Folienschlauch hat aber den entscheidenden Nachteil, daß er nicht "in situ" sterilisierbar ist, da die Folie den dabei auftretenden Innendruck nicht aufnehmen kann. Die Anordnung kann deshalb nur für die Anzucht von mit rein anorganischer Nährlösung versorgten Algen herangezogen werden, bei denen eine Sterilisation nicht notwendig ist. Bei Pflanzenzellkulturen werden jedoch in der Regel organische Nährlösungsbestandteile eingesetzt. Die Sterilistion ist dann nicht zu umgehen. Wie bereits ausgeführt, besteht bei diesem bekannten Behälter keine Möglichkeit, die bei üblichen Fermentern vorgesehenen Einbauten, z.B. eine pH-Sonde, Rührorgane etc. unterzubringen.

Ferner ist aus der US-PS 40 27 427 ein Verfahren und eine Vorrichtung für die Produktion von Mikroorganismen bekannt. Bei der Vorrichtung handelt sich um einen Plastiksack, dessen einzige Öffnung durch eine gasdurchlässige, kreisförmige Filterscheibe verschlossen ist. Die Verbindung des auswechselbaren Filters mit der Plastikfolie wird durch eine kragenförmige Verschraubung bewerkstelligt. Diese Vorrichtung ist für die Produktion kleiner Mengen von Mikroorganismen vorgesehen. Die Installation der bei Fermentern üblichen Einbauten wie Meßsonden, Zu- und Abführleitungen sowie einer Gasverteileinrichtung ist nicht vorstellbar. Die Vorrichtung kann im übrigen ebenfalls nicht "in-situ" sterilisiert werden.

Weiterhin ist durch die GB-PS 85 56 44 ein tonnenförmiger Behälter bekannt, der mit einer Platikfolie ausgekleidet wird. Der die Auskleidung bildende Plastiksack ist mit einer verschraubbaren Öffnung versehen, durch der der Behälter gefüllt oder entleert werden kann.

2

Letztlich ist aus der CH-PS 527 105 ein stapelbarer Kunst- stoffbehälter mit einer Plastiksackeinlagee bekannt, die einen verschließbaren Stutzen besitzt.

Die beiden zuletzt angesprochenen Druckschriften beschreiben also Plastiksäcke, deren Form durch einen äußeren Stützbehälter vorgegeben wird. Die einen Fermenter betreffende Problematik, z.B. der Druckaufnahme während der Sterilisation, wird darin nicht angesprochen.

Der Erfindung liegt die Aufgabe zugrunde, einen Fermenter anzugeben, der bei geringen Herstellkosten leicht zu rei- nigen ist, der die Verwendung genormter Meßsonden und anderer Einbauten wie Rührorgane etc. ermöglicht, und der gegebenenfalls "in situ" sterilisierbar ist.

Eine erfindungsgemäße Lösung dieser Aufgabe ist mit ihren Weiterbildungen in den Ansprüchen gekennzeichnet. Erfindungsgemäß bildet den zylindrischen Fermenter ein zwischen den beiden Stirnplatten eingespannter Folienschlauch. Die den Boden und den Deckel des Fermenters bildenden Stirnplatten ermöglichen weiterhin den Einbau von genormten Meßsonden, Rührorganen, Gaszuführungen, Heizschlangen etc.

Der erfindungsgemäße Fermenter hat durch die bislang bei sterilisierbaren Fermentern noch nicht in Betracht gezogene Verwendung eines Folienschlauches eine Reihe von Vorteilen:

Die Herstellkosten sind niedrig, da außer dem Folienschlauch nur die beiden Stirnplatten benötigt werden, die vergleichsweise einfach beispielsweise aus Edelstahl (Anspruch 16) oder auch aus einem Kunststoffmaterial hergestellt werden können.

Zur Reinigung des Fermenters müssen nur die beiden Stirnplatten gereinigt werden, die in einer normalen Laborspülmaschine gereinigt weren können, während der Folienschlauch durch einen neuen ersetzt wird. Die Kosten für einen neuen Folienschlauch liegen dabei unter den Reinigungskosten für die (aufwendige) Reinigung der Innenwände eines zylindrischen Glas-, Kunststoff- oder Edelstahlteils.

Bei Verwendung eines klar durchsichtigen Folienschlauches ist eine ungehinderte Beobachtung der biologischen Reaktionen sowie ein Einsatz des erfindungsgemäßen Fermenters für photosynthetische Reaktionen möglich. Gleichzeitig können jedoch auch die gleichen Stirnplatten mit einem undurchsichtigen Folienschlauch für einen Fermenter verwendet werden, in dem Experimente ausgeführt werden, bei denen Lichteinfall störend wäre.

Vor allem aber ist durch die in Anspruch 2 angegebene Weiterbildung des erfindungsgemäßen Fermenters eine gefahrlose "in situ-Sterilisation" des erfindungsgemäßen Fermenters möglich: Der Folienschlauch legt sich bei Überdruck im Inneren des Fermenters dicht an den angepaßten Stützmantel an, so daß der Fermenter trotz Verwendung eines Folienschlauches einen Überdruck entsprechend der DECHEMA-Betreibernorm aufnehmen kann. Aber selbst dann, wenn der Folienschlauch einmal bei der Sterilisation reißen sollte, besteht keine Gefahr, da keine Glassplitter "umherfliegen" können.

In den Ansprüchen 3 bis 5 sind vorteilhafte Ausbildungen des Stützmantels gekennzeichnet.

Die Verbindung des Folienschlauches mit den Deckel und Boden des Fermenters bildenden Stirnplatten kann praktisch beliebig erfolgen; bevorzugte Ausbildungen der Verbindung sind in den Ansprüchen 6 bis 9 gekennzeichnet. Dabei hat insbesondere die Ausbildung gemäß 6 den Vorteil, daß der Zusammenbau des Fermenters auch dann einfach ist, wenn in dem Fermenter mit weiteren Folienschläuchen weitere Räume abgeteilt sind (Ansprüche 10 und 11). Die Überwurfplatte des innersten Folienschlauches stellt den "Deckel" für den den innersten Folienschlauch umgebenden Folienschlauch usw. dar.

Eine weitere überraschende Eigenschaft des erfindungsgemä- ßen Folienfermenters ist in Anspruch 13 gekennzeichnet: Trotz des scheinbar labilen Aufbaus mit einer die Zylindermantelfläche bildenden Folie kann ohne weiteres mit hoher Drehzahl von über 3000 U/min gerührt werden.

Die Aufteilung der einzelnen Einbauten auf die obere und untere Stirnplatte kann natürlich entsprechend dem jeweiligen Einsatzzweck erfolgen, wobei in Anspruch 14 eine standardmäßige Aufteilung gekennzeichnet ist.

In den Ansprüchen 15 und 16 sind bevorzugte Materialien für die Stirnplatten und den Folienschlauch gekennzeichnet.

Der erfindungsgemäße Folienfermenter kann - wie in Anspruch 17 beansprucht - auch in ein Reaktionsgefäß aus Stahl eingebaut werden, und dazu dienen, in diesem Reaktionsgefäß einen oder mehrere Reaktionsräume abzuteilen. Dabei können natürlich auch als Wand für einen oder mehrere Reaktionsräume semipermeable Folienschläuche verwendet werden.

Die Erfindung wird nachstehend anhand von Ausführungsbeispielen unter Bezugnahme auf die Zeichnung näher beschrieben, in der zeigen:

Fig. 2 einen erfindungsgemäßen Folienfermenter im Schnitt,

Fig. 3 den zugehörigen Stützmantel,

Fig. 4 ein erstes Ausführungsbeispiel für eine Stirnplatte,

Fig. 5 ein zweites Ausführungsbeispiel für eine Stirnplatte,

Fig. 6 ein Ausführungsbeipiel für einen Stützmantel,

Fig. 7 ein Ausführungsbeipiel eines Fermenter, in dem mit einem weiteren Folienschlauch ein Bereich abgeteilt ist,

Fig. 8 ein ausführungsbeispiel für einen Fermenter, in dem eine Reihe von Reaktionsräumen abgeteilt ist, und

Fig. 9 eine Kombination eines erfindungsgemä- ßen Folien-Fermenters mit einem Stahlgefäß. -

Fig. 2 zeigt einen erfindungsgemäßen zylinderförmigen Fermenter. Den Zylindermantel bildet ein Folienschlauch 1, der mit Hilfe elastischer Gummiringe bzw. O-Ringe 2 mit Stirnplatten 3 und 4 fest verbunden ist.

Der Folienschlauch kann aus einem Polyamid-

Material und die O-Ringe aus Viton (eingetragenes Warenzeichen) bestehen, während die Stirnplatten bei den folgenden Ausführungsbeispielen ein Stahl-Boden 4 bzw. ein Stahl-Deckel 3 sein können.

In der oberen Platte 3 können Meßsonden, wie pH-Sonden, Redox-Sonden, $p_{o2}$-Sonden, Turbidostatsonden 5, die Animpfstutzen, pH-Regulationstutzen, Abluftkühler, eine Zufuhreinrichtung 6 für die Begasung und eventuell ein Überdruckventil untergebracht sein. Die untere Stirnplatte, d.h. der Boden 4 kann eine Kühleinrichtung 7, eine Heizeinrichtung 8, Temperaturmeßfühler, ein Ablaßventil und, wenn notwendig, ein Probeentnahmeventil enthalten. Zu den Einbauten, die in einer der StirnPlatten vorgesehen werden können, zählen beispielsweise auch Gasverteileinrichtungen, Einsteckrohre, Stromstörer und Rührwerk 9.

Der auf diese Weise gebildete Folienschlauch-Fermenter stellt ein exakt zylinderförmiges Gebilde dar, das bei der Sterilisation mit Hilfe eines Stützmantels 10 (Fig. 3) gestützt wird.

Vorteilhafterweise wird, wie bereits ausgeführt, als Folien-Schlauch eine durchsichtige (in Sonderfällen auch eine undurchsichtige) Polyamidfolie mit einer Schmelztemperatur zwischen 170 und 220°C verwendet. Die Foliendicke kann beispielsweise nach der folgenden Auslegungsgleichung bestimmt werden, die für den (hinsichtlich der Auslegung ungünstigeren) Fall berechnet worden ist, daß der Fermenter mit der oberen Stirnplatte aufgehängt ist, daß also keine Abstützung der unteren Stirnplatte existiert, die Kräfte aufnehmen kann:

$$S \; 0.43 * H * D * * g * F / o \quad v$$

Hierbei bedeuten:
S: Wandstärke des Folienschlauchs
H: Höhe des Fermenters
D: Durchmesser des Fermenters
 : Dichte des Fermenterinhalts,
g: Erdbeschleunigung
 v : Zerreißspannung der Folie
F: Sicherheitsfaktor

Mit dieser Auslegungsgleichung erhält man beispielsweise für einen Fermenter mit einer Höhe von 2 m, einem Durchmesser von 200 mm, einem Inhalt mit einer Dichte von 1000 kg/m3 und einem Sicherheitsfaktor von 5 eine Wandstärke von ca 0.2 mm, also eine handelsübliche Foliendicke!

Die Instrumentierung kann wie bei Glasfermentern durchgeführt werden. Der Fermenter kann beispielsweise auch als Blasensäule oder als Schlaufenreaktor betrieben werden.

Die Sterilisation erfolgt in der Regel mit Hilfe des ohnehin in jedem Fermenter eingebauten Temperaturregelsystems. Voraussetzung ist, daß die eingesetzten Heizelemente entsprechend stark ausgelegt sind, um ein Hochheizen des Fermenterinhaltes auf 121°C in einem Zeitraum von etwa 30 Minuten zu ermöglichen. Die Sterilisationstemperatur von 121°C wird über einen Zeitraum von 30 Minuten gehalten. Anschließend wird der Fermenterinhalt auf Betriebsbedingungen abgekühlt. Es ist hierbei nützlich, wenn im unteren Deckel (Boden) des Fermenters eine Kühlmöglichkeit vorgesehen ist. Steht kein Temperaturregler zur Verfügung, so genügt auch eine Heizeinrichtung in Verbindung mit einem am oberen Deckel angebrachten Sicherheitsventil, das auf einem Überdruck von 1,2 bar eingestellt ist (Dampfkochtopfprinzip).

Folienfermenter können auch als Laborreaktoren eingesetzt werden, da sie ähnlich wie Glasreaktoren den Vorteil einer durchsichtigen Außenwand haben. Besonders günstig ist jedoch wegen der geringen volumenbezogenen Fermenterkosten der Einsatz in der technischen Produktion.

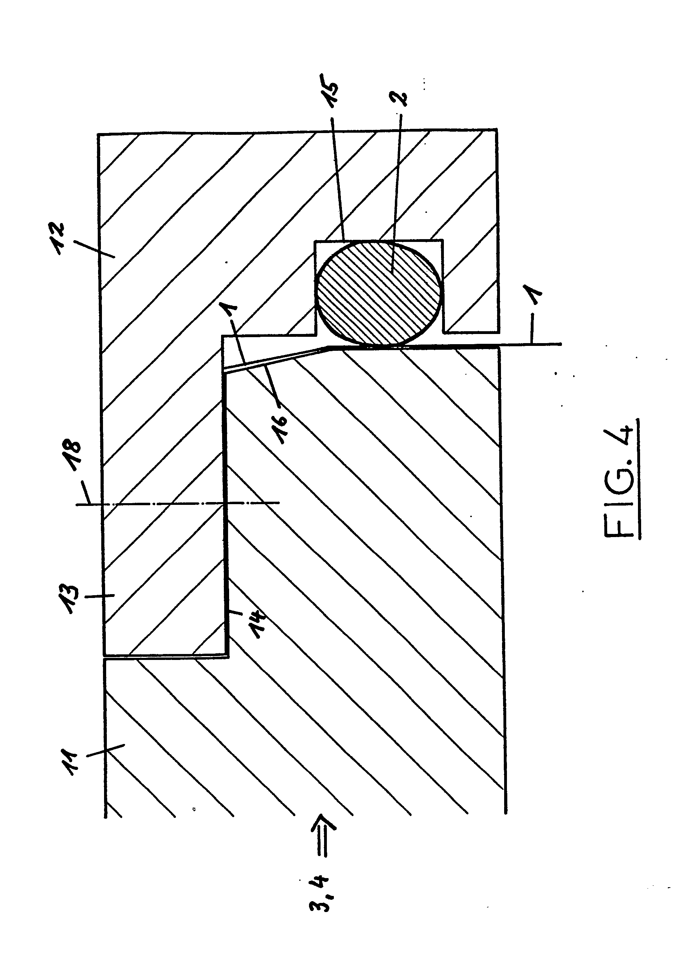

Fig. 4 zeigt ein erstes Ausführungsbeispiel für eine Stirnplatte 3 bzw. 4. Die Stirnplatte besteht aus einem Deckel 11 und einem Haltering 12. Der Haltering 12 weist an seiner Innenseite einen Vorsprung 13 auf, der in eine komplementäre Ausnehmung 14 des Deckels 11 eingreift. An der inneren Mantelfläche des Halterings 12 ist eine ringförmige Nut 15 vorgesehen, in der der O-Ring 2 eingesetzt ist. Um den Haltering 12 ohne Beschädigung des O-Rings 2 auf den Deckel 11 aufsetzen zu können, weist dieser an seiner Oberseite eine Abschrägung 16 auf, die bei dem gezeigten Ausführungsbeispiel ca. 12% beträgt.

Mit der in Fig. 4 dargestellten Stirnplatte 3 bzw. 4 wird der erfindungsgemäße Fermenter wie folgt montiert:

Zunächst wird der Folienschlauch 1 auf den Deckel 11 aufgezogen und genau positioniert. Der Folienschlauch wird hierbei um ca. 4% gegenüber dem Nenndurchmesser des unbelasteten Schlauchs überdehnt. Dabei wird die Folie an einer am Fermenterdeckel 11 vorgesehenen Schnittkante abgeschnitten. Anschließend wird der Haltering 12 mitsamt dem O-Ring 2 über den Deckel 11 geschoben. Aufgrund der aufgebrachten Vorspannung verschiebt sich die Folie bei der Montage nicht. Der Haltering 12 wird am Deckel 11 mit vier Schrauben, von denen in Fig. 4 nur die Achse 18 einer Schraube schematisch dargestellt ist, befestigt.

Die Nut 15, die den O-Ring 2 aufnimmt, ist derart ausgeführt, daß der O-Ring 2 während der Montage derart verformt wird, daß sich der Durchmesser des Rings mit zunächst kreisförmigem Querschnitt in einer Richtung um ca. 12,5% verkleinert. Hierdurch wird die Folie 1 an den Dekkel 11 angedrückt, wodurch der Folienschlauch in axialer Richtung gehalten wird.

Die durch die erfindungsgemäß angegebene Folienhalterung aufgebrachten Haltekräfte sind unerwartet hoch: Bei einem Polyamid-Folienschlauch mit einem Durchmesser von 100 mm und einer Wandstärke von 0,02 mm, der in eine Halterung gemäß Fig. 4 aus Aluminium mit einem O-Ring aus perbunan (eingetragenes Warenzeichen) mit einem Querschnittsdurchmesser von 4 mm derart eingespannt war, daß ein Abschnitt von 9 mm Länge des Folienschlauches innerhalb der Halterung zu liegen kam, lagen die Haltekräfte über der Zugkraft von 50 kp, bei der die Folie etwa 20 cm von der Halterung entfernt abriß.

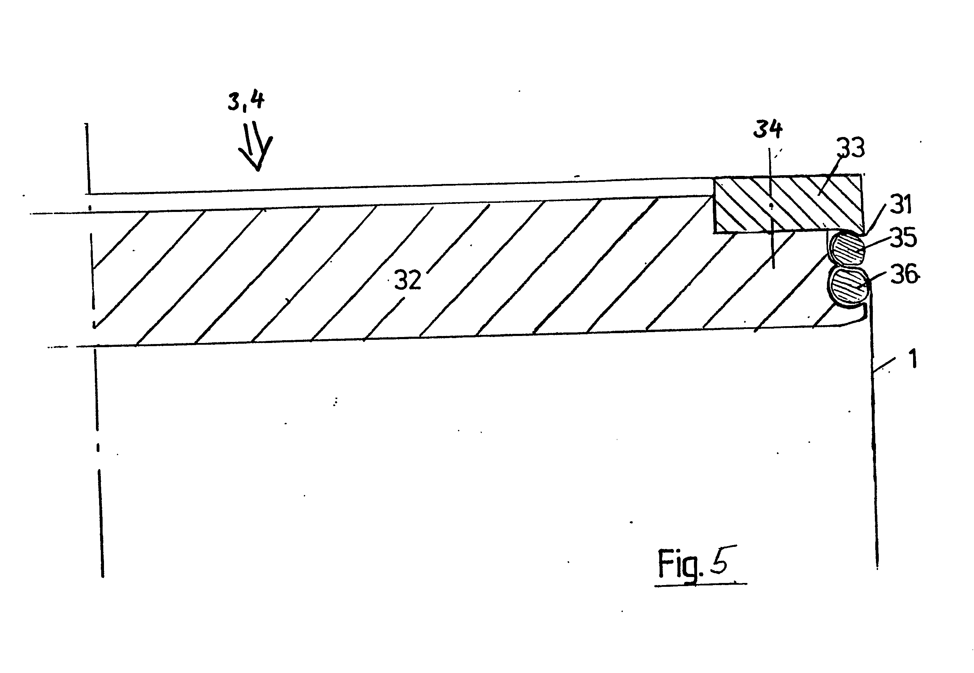

Fig. 5 zeigt ein weiteres Ausführungsbeispiel für eine Stirnplatte.

Die Stirnplatte weist einen Deckel 32 und einen Haltering 33 auf, der in eine ringförmige Umfangsausnehmung des Deckels 32 einsetzbar und in der Ausnehmung mit Schrauben befestigbar ist, von denen in Fig. 5 nur die Achse 34 einer Schraube schematisch dargestellt ist. In der Umfangsfläche des Deckels 32 befinden sich zwei Nuten, in die O-Ringe 35 und 36 eingesetzt sind.

Die eigentliche Abdichtung erfolgt am O-Ring 36, da aufgrund der Kräfte, die durch den hydrostatischen Druck im Reaktionsraum in die folie eingeleitet werden, bzw. aufgrund des Gewichts des gefüllten Folienfermenters bei aufgehängtem Fermenter die Folie 1 an den O-Ring angedrückt wird. Der O-Ring 35 wird durch den Haltering 33 auf den O-Ring 36 gedrückt. Hierdurch wird die dazwischenliegende Folie 1 festgehalten. Der Außendurchmeser des O-Ringes 36 sollte etwas größer sein als der Innendurchmesser des Folienschlauches 1 (im Ausführungsbeispiel ist der Innendurchmesser des Folienschlauches 200 mm, der Außendurchmesser des O-Ringes 205 mm). Auf diese Weise wird der Folienschlauch beim Einbau etwas gedehnt und eine Faltenbildung der Folien an der Dichtfläche zuverlässig verhindert.

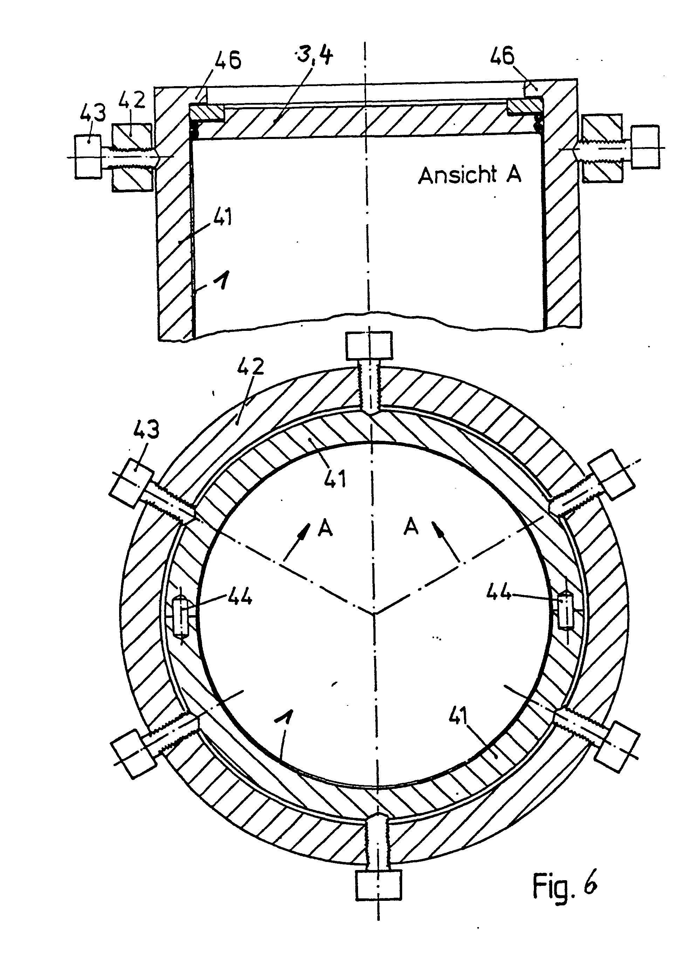

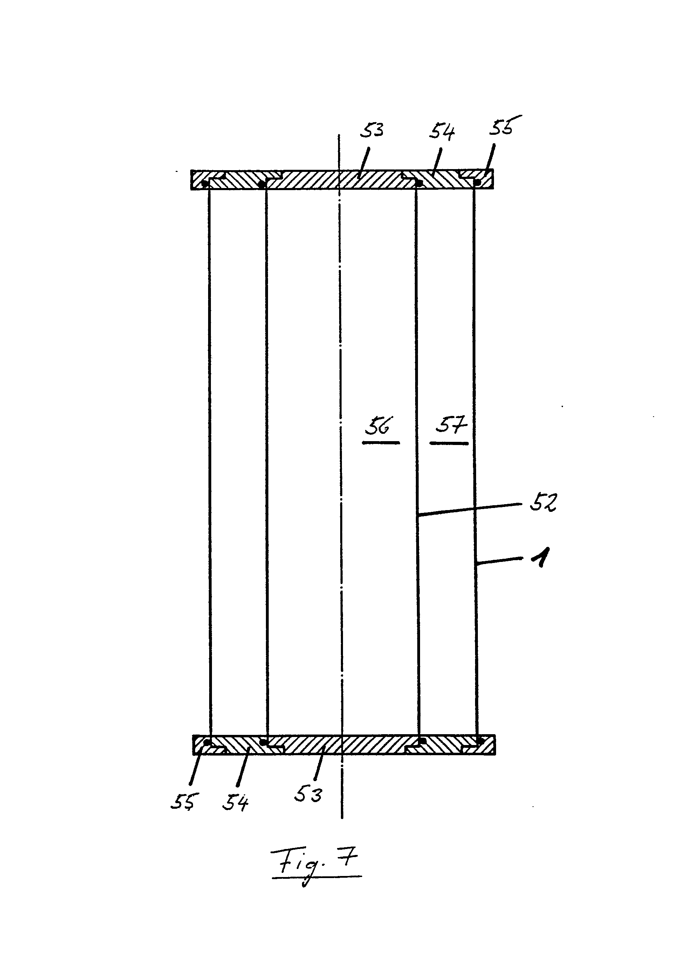

Der Stützmantel für die ''in situ''-Sterilisation ist in Fig. 6 vergrößert gezeigt. Bei der ''in situ''Sterilisation tritt im Inneren des Folienfermenters ein Überdruck von ca. 1,2 bar auf. Deswegen muß während der Sterilisation die Folie 1 abgestützt werden.

Der Stützmantel besteht aus zwei Halbschalen 41, die durch Paßstifte 44 zu einem exakten Zylinderrohr zusammengefügt werden können, so daß sie die Folie 45 eng umschließen. Die beiden Halbschalen 41 können miteinander verschraubt oder durch Halteringe zusammengehalten werden. In diesem Falle werden die Halteringe 42 mit jeweils 6 am Umfang verteilten Schrauben 43 zusammengehalten. Die in axialer Richtung auf die Deckel 40 wirkende Kraft wird durch einen Vorsprung 46 des Stützmantels aufgefangen. Ein zu Testzwecken ausgeführter Stahlstützmantel hat z.B. eine Gesamtlänge von 0,6 m, einen Innendurchmesser von 205 mm für einen Folienschlauch-Durchmesser von 200 mm. Insgesamt sind vier Halteringe 42 angebracht.

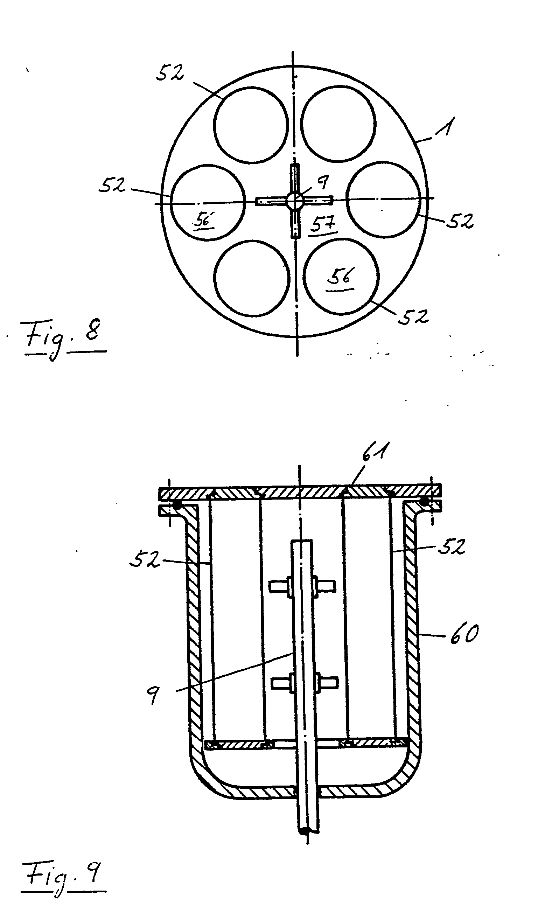

Fig. 7 zeigt ein Ausführungsbeispiel eines Fermenters, in dem mit einem weiteren Folienschlauch ein Bereich abgeteilt ist. Insbesondere mit der Halterung für den Folienschlauch gemäß Fig. 4 ist es möglich, Reaktionssysteme aufzubauen, die mehrere - bevorzugt konzentrisch angeordnete - Folienschläuche enthalten. In Fig. 7 sind zwei konzentrisch angeordnete Folienschläuche 1 und 52 dargestellt. Die Anordnung besteht aus zwei zentralen kreisrunden Deckeln 53, zwei ringförmigen Deckeln 54, die gleichzeitig am Innendurchmesser als Haltering ausgebildet sind, und zwei außenliegenden Halteringen 55.

Eine Vorrichtung der beschriebenen Art kann beispielsweise für die Anzucht tierischer Zellkulturen Verwendung finden. Hierbei kann sich die empfindliche Zellkultur im Raum 56, die mit Nährmedium und Sauerstoff angereicherte Lösung im Raum 57 befinden. Eine Versorgung des Raums 56 mit Nährstoffen sowie eine Entsorgung der anfallenden Stoffwechselprodukte kann über die Folie 52 realisiert werden, wenn diese als semipermeable Membran ausgeführt wird. Solche durchlässigen Folienschlauchmembrane stehen serienmäßig mit verschiedenen Schlauchdurchmessern und einer typischen Wandstärke von 0,02 mm zur Verfügung und werden beispielsweise von der Fa. Enka angeboten. Der entscheidende Vorteil dieser Anordnung besteht darin, daß die Begasung, Thermostatisierung und und Nährstoffeinleitung im Außenraum 57 bewerkstelligt werden kann. Auf diese Weise wird es möglich, die hochempfindlichen Zellkulturen von den damit verbundenen mechanischen Belastungen, Tempeaturgradienten sowie den auftretenden Konzentrationsgradienten zu entlasten.

Fig. 8 zeigt einen Längsschnitt durch einen erfindungsgemäßen Folienfermenter, bei dem mehrere bevorzugt semipermeable Folienschläuche nebeneinander angeordnet werden können. Die äußere Begrenzungswand des Reaktionsraums wird wiederum von einer undurchlässigen Folie 1 gebildet. In dem Reaktionsraum sind mehrere Folienschläuche 52 nebeneinander angeordnet, die Räume beispielsweise für empfindliche Organismen abteilen. Das Versorgungsmedium befindet sich wieder im Außenraum 57. Stellvertretend für eine Vielzahl von möglichen Einbauten ist in Fig. 8 ein Rührwerk 9 dargestellt.

Fig. 9 zeigt ein weiteres Ausführungsbeispiel für einen erfindungsgemäßen Fermenter, dessen Aufbau ähnlich dem in Fig. 8 gezeigten ist. Der äußere Folienschlauch 1 ist jedoch durch einen Stahltopf 60 mit einem Stahldeckel 61 ersetzt, der gleichzeitig einer stabilen Positionierung der einzelnen Folienschläuche 52 dient.

Bei den in den Figuren 8 und 9 gezeigten Ausführungsbeispielen sind die einzelnen Folien in den Deckeln gemäß dem in Verbindung mit Fig. 4 gezeigten Ausführungsbeispiel gehalten.

**Patentansprüche**

1. Sterilisierbarer Fermenter für die Anzucht von Mikroorganismen oder von Zell- bzw. Gewebekulturen in einem zylindrischen Reaktionsraum, den ein zylindrisches Kunststoffteil und zwei Stirnplatten (3, 4) begrenzen, die Einbauten (5, 6, 7, 8) für den Reaktionsraum halten, dadurch gekennzeichnet, daß ein zwischen die Stirnplatten (3, 4) eingespannter Folienschlauch (1) das zylindrische Kunststoffteil bildet.

2. Fermenter nach Anspruch 1, dadurch gekennzeichnet, daß an den Folienschlauch (1) bei der ''in situ''-Sterilisation ein zylindrischer Stützmantel (10) anlegbar ist.

3. Fermenter nach Anspruch 2, dadurch gekennzeichnet, daß der den Folienschlauch (1) bei einer Sterilisation stabilisierende Stützmantel (10) aus zwei Halbschalen (41) besteht, die miteinander verbindbar sind.

4. Fermenter nach Anspruch 3, dadurch gekennzeichnet, daß die Halbschalen (41) miteinander verschraubt sind.

5. Fermenter nach Anspruch 3, dadurch gekennzeichnet, daß die Halbschalen (41) durch Halteringe (42) mit Schrauben (43) zusammengehalten sind.

6. Fermenter nach einem der Ansprüche 1 bis 5, dadurch gekennzeichnet, daß die Stirnplatten (3, 4) aus einem kreisrunden Deckel (11), über den der Folienschlauch (1) gezogen und dessen größter Außendurchmesser etwas größer als der Innendurchmesser des Folienschlauches (1) ist, und einer Überwurfplatte (11) mit einer zumindest teilweise zylindrischen Ausnehmung bestehen, deren Durchmesser etwas größer als der Durchmesser des Folienschlauches (1) ist, und in deren Mantelfläche ein O-Ring (2) eingelegt ist.

7. Fermenter nach Anspruch 6, dadurch gekennzeichnet, daß der kreisrunde Deckel (11) im oberen Teil (16) seiner Mantelfläche abgeschrägt ist.

8. Fermenter nach einem der Ansprüche 1 bis 5, dadurch gekennzeichnet, daß die Stirnplatten (3, 4) zylindrische Platten (32) sind, in deren Mantelfläche eine Aussparung zur Aufnahme von zwei elastischen Ringen (35, 36) vorgesehen sind, die übereinander liegen und zwischen sich unter Einwirkung eines andrückbaren Halterings (33) den Folienschlauch (1) dicht mit den Stirnplatten verbinden.

9. Fermenter nach Anpruch 8, dadurch gekennzeichnet, daß der Außendurchmesser des den Folienschlauch (1) unmittelbar spannenden elastischen Ringes (36) etwas größer ist als der Innendurchmesser des Folienschlauchs (1).

10. Fermenter nach einem der Ansprüche 1 bis 9, dadurch gekennzeichnet, daß in dem Reaktionsraum (57) axial oder außeraxial mittels mindestens eines weiteren Folienschlauchs (52), der axial oder außeraxial zu dem ersten Folienschlauch (1) angeordnet ist, mindestens ein weiterer zylindrischer Raum (56) abgetrennt ist.

11. Fermenter nach Anspruch 10, dadurch gekennzeichnet, daß eine in einem Deckel vorgesehene Verbindungsleitung den Reaktionsraum (57) und den weiteren zylindrischen Raum (56) miteinander verbindet.

12. Fermenter nach Anspruch 10 oder 11, dadurch gekennzeichnet, daß der weitere Folienschlauch (52) eine semipermeable Membran ist.

13. Fermenter nach einem der Ansprüche 1 bis 12, dadurch gekennzeichnet, daß in dem Reaktionsraum ein Rührorgan (9) vorgesehen ist.

14. Fermenter nach einem der Ansprüche 1 bis 13, dadurch gekennzeichnet, daß die obere Stirnplatte (83) mit Meßsonden, Animpfstutzen, pH-Regulationsstutzen, Zufuhreinrichtung für die Begasung, Abluftkühler und gegebenenfalls mit einem Überdruckventil und die untere Stirnplatte mit einer Kühleinrichtung (8), einer Heizeinrichtung (7), Temperaturmeßfühler, einem Ablaßventil, ggf. einem Probeentnahmeventil und einem Einsteckrohr ausgestattet ist.

15. Fermenter nach einem der Ansprüche 1 bis 14, dadurch gekennzeichnet, daß der Folienschlauch (1) aus einem Polyamid besteht.

16. Fermenter nach einem der Ansprüche 1 bis 15, dadurch gekennzeichnet, daß die Deckel (3, 4) aus Edelstahl bestehen.

17. Fermenter nach einem der Ansprüche 1 bis 16, dadurch gekennzeichnet, daß der Fermenter in einem Reaktionsraum (60) aus Stahl eingebaut ist.

**Revendications**

1. Fermenteur stérilisable pour la culture des micro-organismes ou des cultures cellulaires ou de tissu respectivement, dans une chambre de réaction cylindrique définie par un élément en matière plastique et par deux plaques frontales (3, 4) qui supportent des éléments incorporés (5, 6, 7, 8) pour la chambre de réaction, caractérisé en ce qu'une gaine en feuille (1) serrée entre lesdites plaques frontales (3, 4) constitue ledit élément en matière plastique.

2. Fermenteur selon la Revendication 1, caractérisé en ce qu'en stérilisation "in-situ" une chemise de support (10) se peut mettre contre ladite gaine en feuille (1).

3. Fermenteur selon la Revendication 2, caractérisé en ce que ladite chemise de support (10) qui stabilise ladite gaine en feuille (1) au cours d'une opération de stérilisation est constituée par deux semi-coques (41) qu'on peut relier l'une avec l'autre.

4. Fermenteur selon la Revendication 3, caractérisé en ce que lesdites demi-coques (41) sont vissées l'une à l'autre.

5. Fermenteur selon la Revendication 3, caractérisé en ce que ledites demi-coques (41) sont maintenues ensemble au moyen des bagues de fixage (42) à vis (43).

6. Fermenteur selon quelconque des Revendications 1 à 5, caractérisé en ce que lesdites plaques frontales (3, 4) sont constituées par un couvercle circulaire (11) sur lequel est tirée ladite gaine en feuille (1) et dont le diamètre extérieur maximum est un peu plus grand que le diamètre intérieur de ladite gaine en feuille (1), ainsi que par une plaque chapeau (11) à un creux au moins en partie cylindrique dont le diamètre est un peu plus grand que le diamètre de ladite gaine en feuille (1) pendant qu'un O-ring (2) est inséré dans la surface enveloppante de ladite plaque chapeau.

7. Fermenteur selon la Revendication 6, caractérisé en ce que la partie supérieure (16) de ladite surface enveloppante dudit couvercle circulaire (11) est biseautée.

8. Fermenteur selon quelconque des Revendications 1 à 5, caractérisé en ce que lesdites plaques frontales (3, 4) sont des plaques cylindriques (32) dont les surfaces enveloppantes sont pourvues d'un creux à recevoir deux anneaux élastiques (35, 36) qui sont superposés et relient entre eux, sous l'effet d'un anneau de soutien (33) de pression, ladite gaine en feuille (1) jointivement auxdites plaques frontales tube (1).

9. Fermenteur selon la Revendication 8, caractérisé en ce que le diamètre extérieur dudit anneau élastique (36) qui serre la gaine en feuille (1) directement est un peu plus grand que le diamètre intérieur de ladite gaine en feuille (1).

10. Fermenteur selon quelconque des Revendications 1 à 9, caractérisé en ce qu'au moins une chambre cylindrique (56) supplémentaire est séparée dans la chambre de réaction (57) en position axiale ou désaxée, au moyen d'au moins une gaine en feuille (52) additionnelle.

11. Fermenteur selon la Revendication 10, caractérisé en ce qu'un conduit de raccordement pourvu dans un couvercle établit la communication entre ladite chambre de réaction (57) et ladite chambre cylindrique (56) supplémentaire.

12. Fermenteur selon la Revendication 10 ou 11, caractérisé en ce que ladite gaine en feuille (52) additionnelle (52) est un diaphragme semiperméable.

13. Fermenteur selon quelconque des Revendications 1 à 12, caractérisé en ce qu'un élément d'agitation (9) est pourvu dans ladite chambre de réaction.

14. Fermenteur selon quelconque des Revendications 1 à 13, caractérisé en ce que la plaque frontale supérieure (83) est pourvue des têtes de mesure, des tubulures d'inoculation, des tubulures de réglage du pH, des moyens d'alimentation en gaz, d'un refroidisseur de l'air d'évacuation, et en cas de besoin d'une soupape de surpression, pendant que la plaque frontale inférieure est pourvue des moyens de refroidissement (8), d'un dispositif de chauffage (7), des palpeurs pyrométriques, d'une soupape de décharge, ainsi que, si nécessaire, d'une soupape d'échantillonnage et d'une tube à emmancher.

15. Fermenteur selon quelconque des Revendications 1 à 14, caractérisé en ce que ladite gaine en feuille (1) consiste en un polyamide.

16. Fermenteur selon quelconque des Revendications 1 à 15, caractérisé en ce que lesdits couvercles (3, 4) consistent en acier surfin.

17. Fermenteur selon quelconque des Revendications 1 à 16, caractérisé en ce que ledit fermenteur est incorporé dans une chambre de réaction (60) en acier.

## Claims

1. Sterilizable fermenter for cultivation of microorganisms or cell or tissue cultures, respectively, in a cylindrical reaction chamber defined by a cylindrical plastics element and two front plates (3, 4) supporting built-in elements (5, 6, 7, 8) for said reaction chamber, characterized in that a flexible sheeting tube (1) clamped between said front plates (3, 4) constitutes said cylindrical plastics element.

2. Fermenter according to Claim 1, characterized in that with the "in situ" sterilization a cylindrical supporting envelope (10) may be attached to said flexible sheeting tube (1).

3. Fermenter according to Claim 2, characterized in that said supporting envelope (10) which stabilizes said flexible sheeting tube (1) during a sterilization operation is constituted by two shell halves (41) which may be connected to each other.

4. Fermenter according to Claim 3, characterized in that said shell halves (41) are screw-connected to each other.

5. Fermenter according to Claim 3, characterized in that said shell halves (41) are held together by means of holding rings (42) with screws (43).

6. Fermenter according to any of Claims 1 to 5, characterized in that said front plates (3, 4) are constituted by an circular cover (11) over which said flexible sheeting tube (1) is drawn and whose maximum outside diameter slightly exceeds the inner diameter of said flexible sheeting tube (1), as well as by a topping plate (11) with an at least partly cylindrical recess whose diameter slightly exceeds the diameter of said flexible sheeting tube (1) while an O-ring (2) is inserted into the jacket surface of said topping plate.

7. Fermenter according to Claim 6, characterized in that the upper section (16) of the jacket surface of said circular cover (11) is chamfered.

8. Fermenter according to any of Claims 1 to 5, characterized in that the front plates (3, 4) are cylindrical plates (32) whose jacket surfaces are provided with a recess for accomodation of two resilient rings (35, 36) which are sperimposed and attach between them said flexible sheeting tube (1) tightly to said front plates under the action of a press-on holding ring (33).

9. Fermenter according to Claim 8, characterized in that the outside diameter of said resilient ring (36) which directly tightens said flexible sheeting tube (1) slightly exceeds the inner diameter of said flexible sheeting tube (1).

10. Fermenter according to any of Claims 1 to 9, characterized in that at least one additional cylindrical chamber (56) is axially or non-axially separated in said reaction chamber (57) by means of at least one further flexible sheeting tube (52) which is disposed axially or non-axially relative to said first flexible sheeting tube (1).

11. Fermenter according to Claim 10, characterized in that a communication line provided in a cover connects said reaction chamber (57) with said additional cylindrical chamber (56).

12. Fermenter according to Claim 10 or 11, characterized in that said additional flexible sheeting tube (52) is a semi-permeable diaphragm.

13. Fermenter according to any of Claims 1 to 12, characterized in that a stirring element (9) is provided in said reaction chamber.

14. Fermenter according to any of Claims 1 to 13, characterized in that the upper one (83) of said front plates is equipped with measuring probes, inocculation adapters, PH adjustment adapters, feed means for gas supply, exhaust air coolers and, whenever necessary, with a pressure relief valve while the lower front plate is provided with cooling means (8), heating means (7), temperature sensor, a discharge valve and, whenever necessary, with a sampling valve and a plug-in tube.

15. Fermenter according to any of Claims 1 to 14, characterized in that said flexible sheeting tube (1) consists of a polyamide material.

16. Fermenter according to any of Claims 1 to 15, characterized in that said covers (3, 4) consist of stainless steel.

17. Fermenter according to any of Claims 1 to 16, characterized in that said fermenter is incorporated in a reaction chamber (60) made of steel.

Fig. 1

Fig. 2

Fig. 3

FIG.4

EP 0 200 792 B1

Fig. 5

Ansicht A

Fig. 6

53    54    55

56    57

52

1

55    54    53

Fig. 7

Fig. 8

Fig. 9